# EUROPÄISCHE PATENTSCHRIFT

(11) **EP 0 904 751 B1**
(45) Veröffentlichungstag und Bekanntmachung des Hinweises auf die Patenterteilung: **20.08.2003**
(21) Anmeldenummer: 97810721.7
(22) Anmeldetag: 30.09.1997
(51) Int. Cl.: A61F 2/44

(54) **Rohrförmiger Stützkörper zum Überbrücken zweier Wirbel**
Tubular support body for bridging two vertebrae
Corps de soutien tubulaire pour relier deux vertèbres

(43) Veröffentlichungstag der Anmeldung: 31.03.1999
(73) Patentinhaber: Centerpulse Orthopedics Ltd., 6340 Baar (CH)
(72) Erfinder: Studer, Armin, 6312 Steinhausen (CH); Donno, Cosimo, 8400 Winterthur (CH)
(74) Vertreter: Manitz, Finsterwald & Partner Gbr

(56) Entgegenhaltungen:
- WO-A-94/18913
- DE-A- 3 605 630
- DE-A- 4 409 392
- DE-A- 19 504 867
- FR-A- 2 730 158
- US-A- 3 102 536
- US-A- 5 290 312

## Beschreibung

Die Erfindung handelt von einem rohrförmigen Stützkörper zum Ueberbrücken zweier benachbarter Wirbel mit zwei ineinander geführten und mit Durchbrüchen versehenen Käfigen, die jeweils einen Abstützflansch zur äusseren Stirnseite aufweisen.

Stützkörper dieser Art werden im Bereich der Bandscheiben implantiert, wenn die Bandscheiben selbst den Belastungen, die die benachbarten Wirbel auf sie ausüben, nicht mehr standhalten und ein Stützkörper als Platzhalter notwendig wird.

Solche Stützkörper sind häufig als Käfige ausgeführt, die vor dem Implantieren mit autologen Knochenspänen gefüllt werden. Dabei sind die Durchbrüche im Käfig so gewählt, dass das Knochengewebe gefangen ist, dass aber gleichzeitig durch die Durchbrüche nach dem Implantieren eine Versorgung des Knochengewebes stattfindet.

Die Patentschrift DE 195 04 867 zeigt Stützkörper die vor dem Implantieren auf ihre Einbaulänge zusammensteckbar sind. Dies hat den Nachteil, dass die notwendige Einbaulänge im voraus sehr genau vorherbestimmt sein sollte. Der Orthopäde muss daher ein ganzes Arsenal von Bauteilen im Operationssaal haben, um eine passende Grösse zusammenzustellen.

Eine andere Ausführung wird in der EP-A-0 693 274 mit verstellbaren Gewinden gezeigt, die durch Drehen auf eine passende Höhe die Länge eines Stützkörpers festlegen und anschliessend durch Schrauben, die radial in das Gewinde eindringen, gesichert werden. Eine solche Ausführung hat den Nachteil, dass sie wegen dem Schaden der am Gewinde entsteht, eigentlich nur einmal fixierbar ist. Das Erreichen einer bestimmten Vorspannkraft auf den Sicherungsschrauben ist nur über eine plastische Deformation der Gewindegänge am Stützkörper möglich.

Aufgabe der vorliegenden Erfindung ist es mit wenigen Teilen einen mehrfach verstellbaren Stützkörper zu schaffen. Diese Aufgabe wird mit den Kennzeichen vom unabhängigen Anspruch 1 dadurch gelöst, dass ein erster Käfig an seinem Mantel mindestens 3 radial vorstehende Zapfen aufweist, welche in 3 oder ein mehrfaches davon axial verlaufende Zuführkanäle im Mantel des anderen Käfigs einfahrbar sind, um unterschiedlich tiefe Einrastpositionen zu erreichen.

Ein Vorteil der Erfindung besteht darin, dass bereits mit zwei Teilen Stützkörper unterschiedlicher Länge durch blosses Zusammenstecken herstellbar sind und dass keine Gefahr besteht, dass die gesteckte Verbindung sich löst, solange eine Druckkraft in der Hauptbelastungsrichtung durch Bandapparat oder Gewicht vorhanden ist. Ein weiterer Vorteil besteht darin, dass durch definierte Einrastpositionen die Durchbrüche an beiden Käfigen so aufeinander abstimmbar sind, dass die Durchbrüche im Ueberdeckungsbereich miteinander fluchten und somit unabhängig von der Einrastposition eine Versorgung der im Käfig liegenden Knochenspäne und ein Einwachsen stattfinden kann. Ein weiterer Vorteil besteht darin, dass sich keine losen Kleinteile wie Sicherungsschrauben oder Befestigungsschrauben im Operationsfeld befinden.

Weitere vorteilhafte Ausführungen ergeben sich aus den abhängigen Ansprüchen 2 bis 12.

So ist es vorteilhaft die Einrastpositionen am Ende der jeweiligen Zuführkanäle mit einer Klemmung für die Zapfen zu versehen. Dabei kann die Klemmung in radialer Richtung auf die Zapfen erfolgen, wobei die beiden Käfige in radialer Richtung gegeneinander verspannt werden und die Federwirkung der Käfige in sich eine Vorspannkraft erzeugt. Es ist aber auch möglich, die Zapfen an ihrem Umfang durch Verkleinerung der Breite der Zufuhrkanäle in der Einrastposition zu klemmen. Eine elastische Klemmung kommt dabei dadurch zustande, dass die Zuführkanäle im Einrastbereich in Umfangsrichtung nur eine geringe Wandstärke aufweisen und an Aussparungen angrenzen, die ein elastisches Zurückfedern der Wand im Klemmbereich ermöglichen. Die Toleranzen für einen Zapfendurchmesser "D" und für den Klemmdurchmesser "d" des Zuführkanals sollten so gewählt werden, dass 0,01 mm < D-d < 0,2 mm eingehalten wird. Die Klemmung hat den Vorteil, dass bei einer Einstellung vor dem Einbringen des Implantats dieses für das Einbringen wie ein einteiliger Körper gehandhabt werden kann.

Die Stützkörper können an ihren äusseren Enden mit Abschlussstücken versehen werden, die eine schrägliegende äussere Befestigungsebene aufweisen und die in verschiedenen Drehwinkeln auf den Stützkörpern einrastbar sind, um Abweichungen von der Parallelität zwischen zwei benachbarten Wirbeln auszugleichen.

Im folgenden ist die Erfindung anhand von Ausführungsbeispielen beschrieben. Es zeigen:
- Fig.1: Schematisch eine Ansicht von einem aus zwei Käfigen zusammengesteckten, rohrförmigen Stützkörper, wobei für jede Einrastposition ein separater Zuführkanal vorgesehen ist;
- Fig. 2: schematisch eine Ansicht in Explosionsform von zwei Käfigen mit einem Adapterstück, wobei die Einrastpositionen für einen Zapfen von einem Zuführkanal links und rechts abzweigen;
- Fig. 3: schematisch eine Ansicht in Explosionsform von zwei Käfigen, wobei die Einrastpositionen für einen Zapfen auf der Unterseite eines wendelförmigen Zuführkanals angeordnet sind;
- Fig. 4: schematisch eine Seitenansicht des Stützkörpers in Fig. 1 zwischen zwei benachbarten Wirbeln;
- Fig. 5: schematisch eine Seitenansicht des demontierten Stützkörpers in Fig. 1;
- Fig. 6: schematisch einen vergrösserten Ausschnitt aus Fig. 2, der eine der Klemmanordnungen für eine Einrastposition aufzeigt;
- Fig. 7: schematisch einen Schnitt durch ein Adapterstück in Fig. 2, welcher die Verbindung zwischen Adapter und Käfig zeigt; und
- Fig. 8: schematisch im Schnitt verschiedene Adapterstücke zu einem Käfig in Fig. 3.

In den Figuren sind rohrförmige Stützkörper zum Ueberbrücken zweier benachbarten Wirbel gezeigt. Zwei ineinander geführte Käfige 1, 2 sind mit Durchbrüchen versehen und weisen einen Abstützflansch 5 zur äusseren Stirnseite 6 auf. Ein erster Käfig 1 besitzt an seinem Mantel 9a mindestens 3 radial vorstehende Zapfen 7, welche in 3 oder ein mehrfaches davon axial verlaufende Zuführkanäle 8 im Mantel 9b des anderen Käfigs 2 einfahrbar sind, um unterschiedlich tiefe Einrastpositionen 10, 11, 12 zu erreichen.

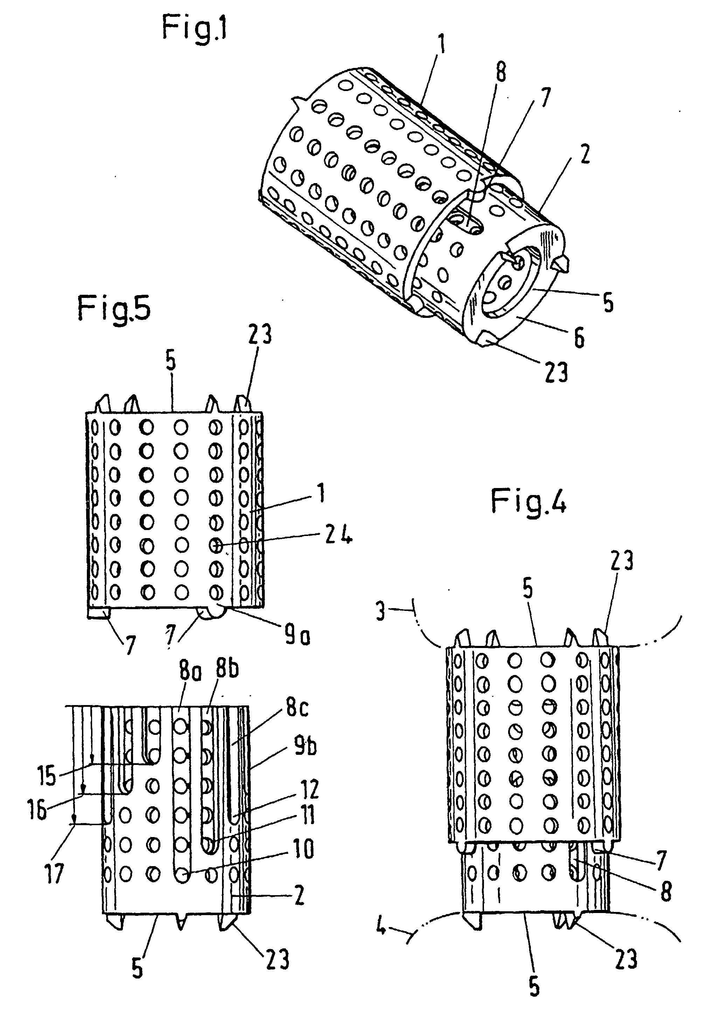

In einem ersten Ausführungsbeispiel Fig. 1, Fig. 4, Fig. 5 sind ein äusserer und ein innerer Käfig 1, 2 aus Titan gezeigt, die auf ihrer äusseren Stirnseite 6 einen Abstützflansch 5 mit vorstehenden Zacken 23 aufweisen. Der innere Käfig 2 besitzt auf seiner äusseren Mantelfläche 9b Zuführkanäle 8, 8a, 8b, 8c, die parallel zur Achse des rohrförmigen Körpers verlaufen und die in unterschiedlichen Tiefen 15, 16, 17 zur Innenseite Einrastpositionen 10, 11, 12 aufweisen. Beide Käfige 1, 2 sind mit Durchbrüchen 24 versehen, die in den verschiedenen Einrastpositionen miteinander fluchten, um genügend grosse Durchbrüche für die Versorgung von eingeschlossenen Knochenspänen zu erhalten. Am äusseren Käfig 1 sind drei radial nach innen vorstehende und um 120° versetzte Zapfen 7 angebracht, die beim Montieren in Zuführkanäle 8a, 8b oder 8c, die ebenfalls um 120° zueinander versetzt sind und eine passende Tiefe 15, 16 oder 17 aufweisen, eingeschoben werden. Mit dem Erreichen einer Einrastposition nimmt die eingefräste Tiefe der Zuführkanäle 8 soweit ab, dass eine radiale Klemmung der Zapfen erfolgt. Zur eigentlichen Klemmung wird die Elastizität der Käfige 1, 2 genutzt, die in den Einrastpositionen 15, 16, 17 in entgegengesetzter Richtung radial deformiert werden. Das Spiel zwischen der äusseren Mantelfläche 9b vom inneren Käfig 2 und der inneren Mantelfläche vom äusseren Käfig 1 ist so gross gehalten, dass sich die Mantelflächen in der Ebene der drei Klemmstellen nicht berühren. Die so entstandene Federkennlinie verläuft flacher als bei Berührung und stellt weniger hohe Anforderungen an die Masstoleranzen, um eine bestimmte Klemmkraft zu erreichen. Die Klemmung der beiden Käfige ist lösbar, sodass jede der möglichen Tiefen 15, 16, 17 im Operationssaal einstellbar ist, um den Stützkörper anschliessend mit Knochenspänen zu stopfen und zwischen zwei auseinandergespreizten Wirbeln 3, 4 einzusetzen. Wenn zwischen den beiden Wirbeln eine Bandscheibe noch vorhanden ist, muss diese seitliche Aussparungen zum Einsetzen der beispielsweise paarweise verwendeten Stützkörper aufweisen.

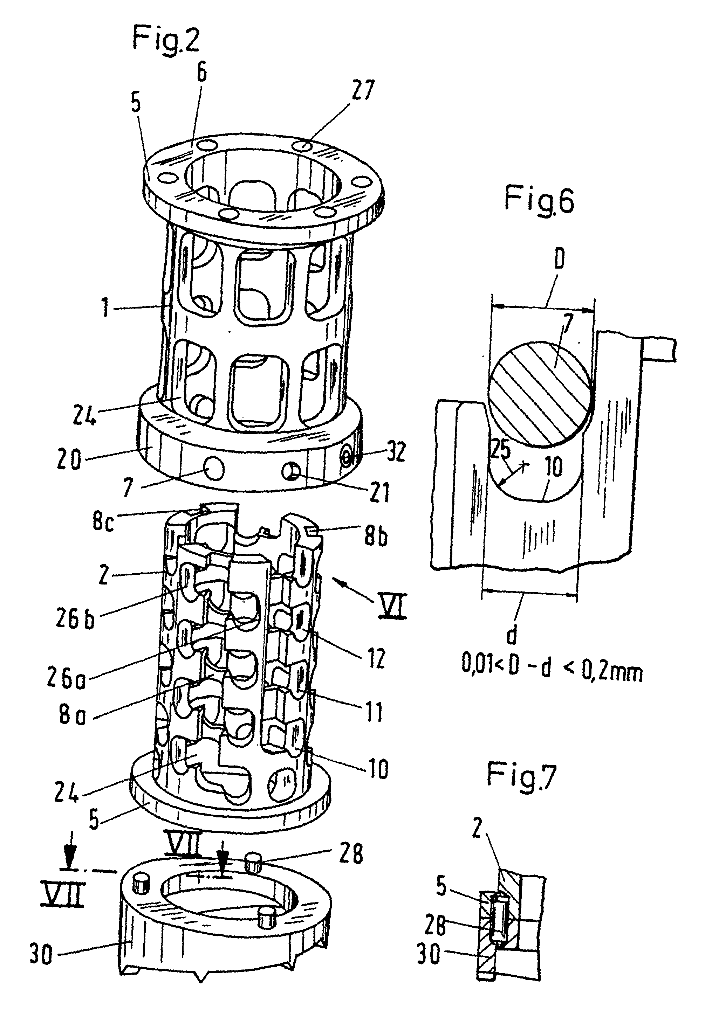

Ein weiteres Ausführungsbeispiel Fig. 2, Fig. 6, Fig. 7 zeigt einen äusseren Käfig 1 mit einem Abstützflansch 5, der Aufnahmebohrungen 27 aufweist, und mit einem Ring 20, in welchem drei Zapfen 7 um 120° versetzt und nach innen radial vorstehend eingelassen sind. In dem Ring 20 sind radiale Bohrungen 21 für Verstellwerkzeuge angebracht und fakultativ ist eine versenkte Sicherungsschraube 32 eingesetzt. Aussparungen 24 im äusseren Käfig 1 sind so gross gewählt, dass unabhängig von der Einrastposition die Durchbrüche 24 des inneren Käfigs 2 für den kleinsten Querschnitt massgebend sind.

Der innere Käfig 2 besitzt auf seiner äusseren Seite ebenfalls einen Abstützflansch 5 mit Aufnahmebohrungen für eventuelle Adapterstücke 30. Drei Zuführkanäle 8a, 8b, 8c sind um 120°zueinander versetzt. Von jedem dieser Zuführkanäle 8a, 8b, 8c sind nach links und rechts Einrastpositionen 26a, 26b erreichbar, die in Fig. 6 näher beschrieben sind. Ein Zapfen 7 macht aus dem Zuführkanal 8a, 8b, 8c zunächst eine Drehbewegung in der Horizontalen und wird dann in einem Uebergangsbereich in die eigentliche Einrastposition 10 abgesenkt. Im Zuführkanal und im Uebergangsbereich ist die Weite vom Kanal grösser als der Durchmesser D vom Zapfen 7. Erst in der Einrastposition ist die Kanalweite d kleiner und genügt der Bedingung 0,01 mm < D-d < 0,2 mm. Das genaue Mass D-d für die Vorspannung in der Einrastposition hängt davon ab, um wieviel die Kanalwände elastisch nachgeben können. In der Einrastposition 10 liegt der Zapfen 7 an einem unteren Punkt und an zwei seitlichen Punkten auf. Ein Schmiegeradius 25 zwischen der Horizontalen und Vertikalen ist kleiner als der Radius vom Zapfen 7, damit im Bereich vom Schmiegeradius ein Spiel vorhanden ist und damit definierte Kraftangriffspunkte gegeben sind.

In Fig. 2 sind am inneren Käfig 2 drei weitere, um 120° versetzte Zuführkanäle angebracht von, denen nur nach einer Seite Einrastpositionen 10, 11, 12 abzweigen, die ihrerseits Zwischenstufen zu den beidseitig abzweigenden Einrastpositionen 26a, 26b darstellen.

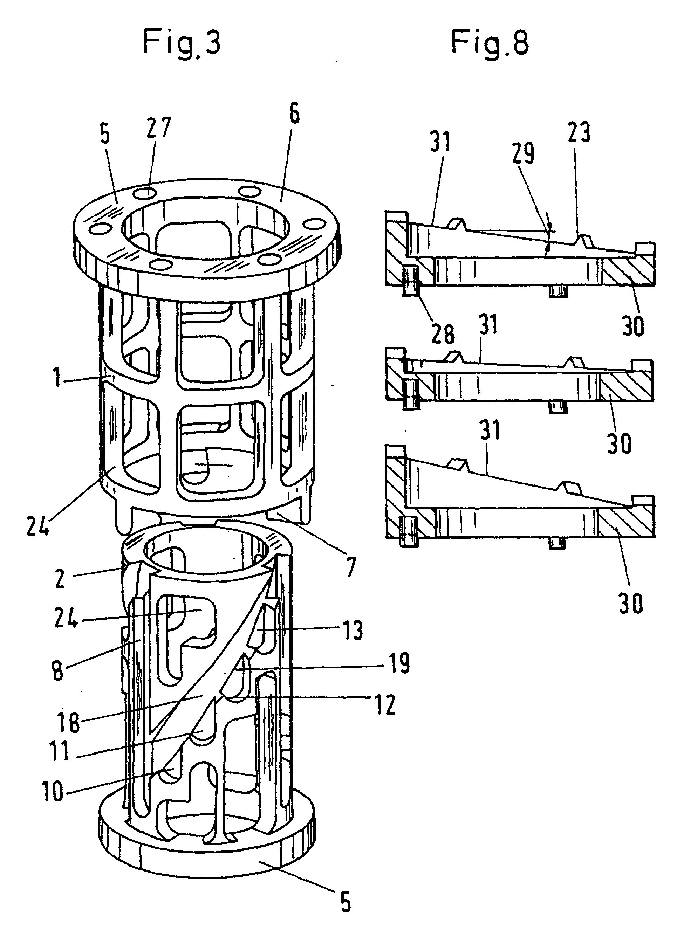

Die Bohrungen 27 in den Flanschen 5 sind um 120° versetzt. Passend dazu sind Adapterstücke 30 vorgesehen, die mit eingepressten Stiften 28 in den Flanschen 5 verankerbar sind. Es sind mehrere Adapterstücke 30 mit verschiedenen Neigungswinkeln 29, wie in Fig. 8 gezeigt, möglich.

Fig. 3 und Fig. 8 zeigen ein weiteres System bei dem der innere Käfig 2 drei um 120° versetzte Zuführkanäle 8 besitzt, die jeweils durch einen weiteren Zuführkanal in Form einer Wendel 18 mit dem benachbarten Zuführkanal verbunden sind. An der Unterseite 19 dieser wendelförmigen Zuführkanäle 18 zweigen zwangsläufig in verschiedenen Tiefen Einrastpositionen 10, 11, 12 ab, in denen die Zapfen 7 nach einem der vorher beschriebenen Verfahren radial 13 oder tangential geklemmt werden. Wenn das Füllen der Käfige mit Knochenspänen zum Teil nach dem Einsetzen am äusseren Käfig 1 erfolgen kann, hat diese Lösung den Vorteil, dass die Käfige 1, 2 beim Einsetzen zwischen zwei Wirbel in ihrer niedrigsten Form über die Zuführkanäle 8 zusammengesteckt sein können und dass eine passende Einrastposition 10, 11, 12 nach dem Einführen zwischen die beiden Wirbel durch eine Verdrehung der Zapfen 7 entlang der Wendel 18 erreichbar ist. Zusammen mit verschiedenen Adapterstücken 30 besteht eine gute Anpassung in der Neigung 29 und im Abstand der Abstützflächen 31 zu den Wirbeln. Die Abstützflächen 31 können mit verschiedenen Zacken 23 und Rücksprüngen bestückt sein, um eine Verankerung im Wirbel zu bewirken.

## Patentansprüche

1. Rohrförmiger Stützkörper zum Ueberbrücken zweier benachbarter Wirbel (3, 4) mit zwei ineinander geführten und mit Durchbrüchen versehenen Käfigen (1, 2), die jeweils einen Abstützflansch (5) zur äusseren Stirnseite (6) aufweisen, **dadurch gekennzeichnet, dass** ein erster Käfig (1) an seinem Mantel (9a) mindestens 3 radial vorstehende Zapfen (7) aufweist, welche in 3 oder ein mehrfaches davon axial verlaufende Zuführkanäle (8) im Mantel (9b) des anderen Käfigs (2) einfahrbar sind, um unterschiedlich tiefe Einrastpositionen (10, 11, 12) zu erreichen.

2. Stützkörper nach Anspruch 1, **dadurch gekennzeichnet, dass** der Uebergang vom Zuführkanal (8) in die Einrastposition (10, 11, 12) mit einer radialen Verschiebung (13) vom Kanalgrund (14) zu den Zapfen (7) hin versehen ist, um eine Klemmung der Zapfen (7) beim Einrasten zu bewirken.

3. Stützkörper nach Anspruch 1 oder 2, **dadurch gekennzeichnet, dass** für jeden Zapfen (7) und für jede Einrasttiefe (10, 11, 12) ein separater Zuführkanal (8a, 8b, 8c) vorgesehen ist.

4. Stützkörper nach Anspruch 1 oder 2, **dadurch gekennzeichnet, dass** für jeden Zapfen (7) ein einzelner Zuführkanal (8) mit seitlich durch Drehung erreichbaren Einrastpositionen (10, 11, 12) in unterschiedlicher Tiefe (15, 16, 17) vorgesehen ist.

5. Stützkörper nach Anspruch 4, **dadurch gekennzeichnet, dass** die Einrastpositionen (10, 11, 12) an einem Zuführkanal (8) wechselseitig versetzt sind.

6. Stützkörper nach Anspruch 1 oder 2, **dadurch gekennzeichnet, dass** für jeden Zapfen (7) ein einzelner Zuführkanal (8) vorgesehen ist, wobei der Zuführkanal in Form einer Wendel (18) verläuft, auf deren Unterseite (19) in verschiedenen Tiefen Einrastpositionen (10, 11, 12) abzweigen.

7. Stützkörper nach einem der Ansprüche 4 bis 6, **dadurch gekennzeichnet, dass** der äussere Käfig (1) einen Ring (20) mit radialen Bohrungen (21) für das Einsetzen eines Verstellwerkzeuges aufweist.

8. Stützkörper nach einem der Ansprüche 1 bis 7, **dadurch gekennzeichnet, dass** der äussere Käfig (1) eine Gewindebohrung zum Sichern des inneren Käfigs (2) mit einer Schraube (32) nach dem Erreichen einer der Einrastpositionen (10, 11, 12) aufweist.

9. Stützkörper nach einem der Ansprüche 1 bis 8, **dadurch gekennzeichnet, dass** der erste Käfig (1) zusätzlich zu den vorstehenden Zapfen (7), weitere in der Richtung der Zuführkanäle zurückversetzte Zapfen (22) aufweist, um zur Erhöhung der Stabilität mit diesen weiteren Zapfen (22) Einrastpositionen (10, 11, 12) in einer anderen Ebene zu erreichen.

10. Stützkörper nach einem der Ansprüche 1 und 3 bis 9, **dadurch gekennzeichnet, dass** die Einrastpositionen (10, 11, 12) gegenüber den Zapfen (7) ein seitliches Untermass 0,01 < D-d < 0,2 mm aufweisen.

11. Stützkörper nach einem der Ansprüche 1 bis 10, **dadurch gekennzeichnet, dass** die Abstützflansche (5) Bohrungen (27) aufweisen, in denen Zapfen (28) von Adapterstücken (30) in unterschiedlichen Winkelpositionen einrastbar sind.

12. Stützkörper nach Anspruch 11, **dadurch gekennzeichnet, dass** Adapterstücke (30) mit unterschiedlich zu den Flanschen (5) geneigten (29) äusseren Abstützflächen (31) einsetzbar sind.

## Claims

1. Tubular support body for bridging two adjacent vertebrae (3, 4) comprising two cages (1, 2) which are guided one within the other, which are provided with cut-outs and each of which has a support flange (5) at the outer end surface (6), **characterised in that** a first cage (1) has at least three radially projecting spigots (7) at its jacket (9a) which can be introduced into three axially extending lead-in channels (8) or a multiple thereof in the jacket (9b) of the other cage (2) in order to be able to reach latch-in positions (10, 11, 12) of different depths.

2. Support body in accordance with claim 1, **characterised in that** the transition from the lead-in channel (8) into the latch-in position (10, 11, 12) is provided with a radial displacement (13) from the base of the channel (14) in the direction towards the spigots (7) in order to effect a clamping of the spigots (7) during the latching in.

3. Support body in accordance with claim 1 or claim 2, **characterised in that** a separate lead-in channel (8a, 8b, 8c) is provided for each spigot (7) and for each latch-in depth (10, 11, 12).

4. Support body in accordance with claim 1 or claim 2, **characterised in that** an individual lead-in channel (8) with latch-in positions (10, 11, 12) at different depths (15, 16, 17) which can be reached laterally by rotation is provided for each spigot (7).

5. Support body in accordance with claim 4, **characterised in that** the latch-in positions (10, 11, 12) at a lead-in channel (8) are mutually offset.

6. Support body in accordance with claim 1 or claim 2, **characterised in that** an individual lead-in channel (8) is provided for each spigot (7), with the lead-in channel extending in the shape of a spiral or helix (18), with latch-in positions (10, 11, 12) branching off from the lower side (19) of the helix at different depths.

7. Support body in accordance with any one of claims 4 to 6, **characterised in that** the outer cage (1) has a ring (20) with radial bores (21) for the insertion of an adjusting tool.

8. Support body in accordance with any one of claims 1 to 7, **characterised in that** the outer cage (1) has a threaded bore for securing the inner cage (2) with a screw (32) after one of the latch-in positions (10, 11, 12) has been reached.

9. Support body in accordance with any one of claims 1 to 8, **characterised in that** the first cage (1) has, in addition to the projecting spigots (7), further spigots (22) which are set back in the direction of the lead-in channels in order to be able to reach latch-in positions (10, 11, 12) in a different plane with these further spigots (22) for increasing the stability.

10. Support body in accordance with any one of claims 1 and 3 to 9, **characterised in that** the latch-in positions (10, 11, 12) have a lateral undersize 0.01 < D - d < 0.2 mm with respect to the spigots (7).

11. Support body in accordance with any one of claims 1 to 10, **characterised in that** the support flanges (5) have bores (27) in which pins (28) of adapter pieces (30) can be latched at different angular positions.

12. Support body in accordance with claim 11, **characterised in that** adapter pieces (30) can be inserted having outer support surfaces (31) which are differently inclined (29) from the flanges (5).

## Revendications

1. Corps de soutien tubulaire pour relier deux vertèbres voisines (3, 4) avec deux cages (1, 2), insérées l'une dans l'autre et dotées de perforations, qui présentent respectivement un bourrelet d'appui (5) sur la face frontale externe (6), **caractérisé en ce qu'**une première cage 1 possède sur sa paroi 9a au moins 3 tenons proéminents sue le plan radial (7) qui peuvent être rentrés dans 3 canaux d'acheminement ou dans une multiplicité de canaux d'acheminements (8) s'étendant sur le plan axial dans le corps (9b) de l'autre cage (2), pour obtenir des positions d'encliquetage de profondeurs différentes 10, 11, 12.

2. Corps de soutien selon la revendication 1, **caractérisé en ce que** le passage vers le canal d'acheminement (8) en position d'encliquetage (10, 11, 12) comprend un décalage radial (13) du fond du canal (14) jusqu'aux tenons (7) pour provoquer un blocage des tenons (7) lors de l'encastrement.

3. Corps de soutien selon la revendication 1 ou 2, **caractérisé en ce que**, pour chaque tenon (7) et pour chaque profondeur d'encliquetage (10, 11, 12), un canal d'acheminement séparé (8a, 8b, 8c) est prévu.

4. Corps de soutien selon la revendication 1 ou 2, **caractérisé en ce que** pour chaque tenon (7), un canal d'acheminement unique (8) est prévu avec des positions d'encliquetage que l'on peut atteindre latéralement par rotation, à des profondeurs diverses (15, 16, 17).

5. Corps de soutien selon la revendication 4, **caractérisé en ce que** les positions d'encliquetage (10, 11, 12) sont déplacées réciproquement sur un canal d'acheminement (8).

6. Corps de soutien selon la revendication 1 ou 2, **caractérisé en ce que**, pour chaque tenon (7) est aménagé un seul canal d'acheminement (8), le canal d'acheminement s'étendant sous la forme d'une spirale (18) sur la face inférieure (19) à partir de laquelle bifurquent dans différentes profondeurs, les positions d'encliquetage (10, 11, 12).

7. Corps de soutien selon l'une quelconque des revendications 4 à 6, **caractérisé en ce que** la cage externe (1) présente une bague (20) avec des alésages radiaux (21) pour la mise en place d'un outil de réglage.

8. Corps de soutien selon l'une quelconque des revendications 1 à 7, **caractérisé en ce que** la cage externe (1) présente une perforation filetée pour fixer la cage interne (2) à l'aide d'une vis (32) une fois que les positions d'encliquetage (10, 11, 12) sont atteintes.

9. Corps de soutien selon l'une quelconque des revendications 1 à 8, **caractérisé en ce que** la première cage (1) présente en plus des tenons proéminents (7), d'autres tenons (22) décalés dans la direction des canaux d'acheminement pour atteindre un autre niveau, afin d'augmenter la stabilité avec cet autre tenon (22).

10. Corps de soutien selon l'une quelconque des revendications 1 et 3 à 9, **caractérisé en ce que** les positions d'encastrement (10, 11, 12) présentent en face des tenons (7) une dimension inférieure latérale telle que 0,01 < D-d < 0,2 mm.

11. Corps de soutien selon l'une quelconque des revendications 1 à 10, **caractérisé en ce que** les bourrelets d'appui (5) présentent des alésages (27) dans lesquelles des tenons (28) des pièces d'adaptation (30) peuvent être encliquetés dans des positions d'angles différents.

12. Corps de soutien selon la revendication 11, **caractérisé en ce que** les pièces d'adaptation (30) peuvent être utilisées avec des surfaces d'appui externes (5) orientées différemment (29) par rapport aux bourrelets (31).
